# EUROPEAN PATENT SPECIFICATION

(11) **EP 4 009 853 B1**
(45) Date of publication and mention of the grant of the patent: **22.03.2023**
(21) Application number: 20739685.4
(22) Date of filing: 14.07.2020
(51) Int. Cl.: A61B 5/00

(54) **A DERMATOSCOPY DEVICE FOR CHECKING SKIN LESIONS**
DERMATOSKOPIEVORRICHTUNG ZUR ÜBERPRÜFUNG VON HAUTLÄSIONEN
DISPOSITIF DE DERMATOSCOPIE PERMETTANT DE VÉRIFIER DES LÉSIONS CUTANÉES

(30) Priority: 06.08.2019 EP 19382687
(43) Date of publication of application: 15.06.2022
(73) Proprietor: DERMAVISION SOLUTIONS, SL, 17480 Roses (Girona) (ES)
(72) Inventor: CAMPMOL AMETLLER, Enric, 17480 Roses (ES); RICART GELI, Narcís, 17480 Roses (ES)
(74) Representative: Segui Quetglas, Margalida
(86) International application number: PCT/EP2020/069850
(87) International publication number: WO 2021/023481

(56) References cited:
- EP-A1- 3 351 162
- US-A1- 2010 232 773
- US-A1- 2013 053 701
- KOROTKOV KONSTANTIN ET AL: "A New Total Body Scanning System for Automatic Change Detection in Multiple Pigmented Skin Lesions", IEEE TRANSACTIONS ON MEDICAL IMAGING, IEEE SERVICE CENTER, PISCATAWAY, NJ, US, vol. 34, no. 1, 1 January 2015 (2015-01-01), pages 317-338, XP011568811, ISSN: 0278-0062, DOI: 10.1109/TMI.2014.2357715 [retrieved on 2014-12-24]

## Description

### Technical Field

The present invention is directed, in general, to the dermatology field. In particular, the invention relates to a dermatoscopy device, for checking skin lesions.

### Background of the Invention

International patent application WO-A1-2013169326 discloses an imaging station/booth for automated total body imaging having a small footprint and capable of quickly, efficiently, effectively, and consistently capturing multiple body images of a user or patient over time with minimal assistance from medical staff.

International patent application WO-A1-2018160720 relates to three-dimensional image capture, particularly with the use of polarized light. The capture of three-dimensional (3D) images of a subject, such as a human, typically entails the use of multiple cameras and light sources, arranged around the subject, that are activated simultaneously while the subject remains still. The number of cameras and light sources required depends in direct relation on the size of the area to be imaged. International patent application WO-A1-2010107467 discloses an imaging station and method for repeatable alignment of images. In some embodiments, the imaging station includes a rotatable stage and a monostand. The rotatable stage may include a structure to support sets of handles for positioning of a subject's hands for at least some of the body poses. A camera is positioned on the monostand for capturing images of the subject and is configured to allow the camera to be repeatably positioned with respect to the rotatable stage for each body pose.

US patent US-B1-9980649 discloses a scan head for scanning skin. The scan head includes a frame and a camera coupled to the frame. A controllable probe is coupled to the frame and is configured to change an orientation of hair on the skin to be examined and imaged with the camera.

International patent application WO-A1-2016188659 discloses a method and a device for producing a series of images of an object with respective predetermined imaging parameters by means of a partially automated imaging system, comprising a database, an imaging unit, a computing unit, a storage unit and an output unit, wherein it is ensured that a manually to be adjusted imaging parameter corresponds to an actual imaging parameter.

Scientific document "A new total body scanning system for automatic change detection in multiple pigmented skin lesion", K. Korotkov et al. discloses a new photogrammetry-based total body scanning system that allows for skin surface image acquisition using cross-polarized light. The system is equipped with 21 high-resolution cameras and a turntable. This scanner automatically acquires a set of overlapping images, covering 85%-90% of the patient's skin surface. These images are used for the automated mapping of PSLs and their change estimation between explorations. The scanner can be applied for automated mapping and temporal monitoring of multiple lesions.

In the known dermatoscopy imaging devices/stations, a single dermatoscopic image is acquired invasively, i.e. not acquired from a distance at least greater than 1-3 mm. Moreover, the known imaging devices/stations do not allow operating in fully controlled lighting conditions using a single image acquisition system comprising different types of cameras.

New imaging devices and method for checking skin lesions in a location with controlled light and image acquisition are therefore needed.

### Description of the Invention

The object of this invention is fulfilled by a dermatoscopy device with the characteristics of claim 1.

The present invention proposes, according to one aspect, a dermatoscopy device for checking skin lesions. Similar to the devices known in the field, the proposed dermatoscopy device comprises:
- an enclosed chamber to accommodate a user/patient to be examined;
- a displacement element to be moved on axes x, y, z inside the enclosed chamber;
- an image acquisition system attached on a support of the displacement element to acquire images from different body regions of said user;
- lighting elements;
- a computing unit, operatively connected to said displacement element and to said image acquisition system, to process said acquired images.

Unlike the known devices in the field, the image acquisition system of the proposed device comprises two different types of cameras. A first type of the cameras includes a stereoscopic camera or two cameras configured to work/operate as a stereoscopic camera. A second type of the cameras comprises a dermatoscopic camera (e.g. a long-range dermatoscopic camera). The camera(s) of the first type is/are configured to operate simultaneously (if there are more than one) and independently of the dermatoscopic camera.

The lighting elements comprise at least two lighting elements each one attached on an extreme of the support of the displacement element. Thus, in the proposed device, the images are always acquired under controlled lighting conditions.

Moreover, in the proposed device the cited processing comprises analyzing, for each body region, at least one image acquired by the camera(s) of the first type; executing a scanning algorithm that detects skin structures (e.g. freckles, skin marks, among others) in said image and that checks whether said skin structures fulfill a given criteria; upon a given skin structure of a given body region is determined as fulfilling the given criteria, executing a motion algorithm that automatically moves the displacement element to the given body region; and providing a triggering order to the image acquisition system. Therefore, as a result, the dermatoscopic camera can acquire, at or from a determined distance, at least one image of the given skin structure.

Therefore, present invention provides a medical device that provides automated and accurate clinical and dermatoscopic images of a patient's skin. Moreover, the dermatoscopic images of individual skin lesions are acquired in a standardized way (controlled light and acquisition distance). In addition, the present invention provides the physician/doctor with the necessary information such as the color, dimension, shape, asymmetry and evolution of each of the skin structures in order to make an accurate diagnosis.

The camera(s) of the first type can be a passive stereo camera(s), an active stereo camera(s) with a structured light source or a laser, a time-of-flight (TOF) camera(s) or the combination of a LIDAR and a single camera.

In an embodiment, the determined distance is comprised in a range between 100-1000 mm from the user, particularly between 300-600 mm. Therefore, the acquisition is performed non-invasively.

In an embodiment, the displacement element is arranged on a guiding railway of a guiding system. The guiding system is arranged on an interior wall of the enclosed chamber. Particularly, the guiding system comprises two vertical guide members.

In another embodiment, each of the lighting elements comprises a light emitting diode (LED). The lighting elements also include a light confinement chamber in order to only illuminate a given zone when the image/s is/are acquired. Furthermore, the lighting elements also include a polarization filter, which is 90° out of phase with respect to a polarization filter of the dermatoscopic camera in order to generate a cross polarization. The lighting elements in an embodiment are separated a certain distance on said support in order to operate at a given angle (between 35 and 55º).

In yet another embodiment, the proposed device also has a positioning unit to indicate a position in which the user has to be placed inside the enclosed chamber. For example, the position unit can be a floor display or even a loudspeaker which guides the user to the different positions. Alternatively, the positioning unit can be configured to automatically move the user towards the interior wall of the enclosed chamber. The displacement element in some embodiments can also include an articulable or pivotable element that allows the displacement element to be moved in multiple degrees of freedom.

Embodiments not part of the present invention also propose, a method for checking skin lesions. The method comprises a) accommodating a user to be examined within an enclosed chamber of a dermatoscopy device; b) moving, via a displacement element of the dermatoscopy device, an image acquisition system of the dermatoscopy device towards different body parts of a given body side of the user; c) acquiring, by at least one camera of a first type of the image acquisition system, one or more images of the different body parts of the user, the at least one camera of the first type comprising a stereoscopic camera or two cameras operating as a stereoscopic camera, and the one or more images being stereoscopic images that are acquired under controlled lighting conditions; d) analyzing, by a computing unit, the acquired stereoscopic images by executing a scanning algorithm that checks whether skin structures included in the stereoscopic images fulfill a given criteria; e) upon a given skin structure of a given body region is determined as fulfilling the given criteria, executing, by the computing unit, a motion algorithm that automatically moves the displacement element towards the given body region; f) in response to a triggering order from the computing unit, acquiring, by a camera of a second type of the image acquisition system, which is a dermatoscopic camera, at/from a determined distance, at least one dermatoscopic image of the given skin structure; and g) providing the acquired at least one dermatoscopic image using the computing unit.

According to a proposed method not part of the invention, said step b) is performed for all the body sides of the user.

In an embodiment, the cited criteria is based on an ABCD rule of dermatoscopy based on the criteria asymmetry, A, border, B, color, C, and differential structure, D, of the skin structures.

In another embodiment, the scanning algorithm comprises a neural network. The neural network is particularly trained to automatically recognize if the skin structure looks like a benign or malign structure. In other embodiments, the scanning algorithm comprises a decision tree-based algorithm and/or a support vector machine.

### Brief Description of the Drawings

The previous and other advantages and features will be more fully understood from the following detailed description of embodiments, with reference to the attached figures, which must be considered in an illustrative and non-limiting manner, in which:
Fig. 1 illustrates the proposed dermatoscopy device for checking skin lesions, according to an embodiment of the present invention.
Fig. 2 illustrates in more detail the image acquisition system, lighting elements and displacement element of the embodiment of Fig. 1.
Fig. 3 illustrates another embodiment of the image acquisition system, lighting elements and displacement element.
Fig. 4 schematically shows an example of the different type of images that can be acquired with the present invention.
Fig. 5 is a flow chart showing the steps executed by the computing unit to check skin lesions, according to an embodiment of the present invention.

### Detailed Description of Preferred Embodiments

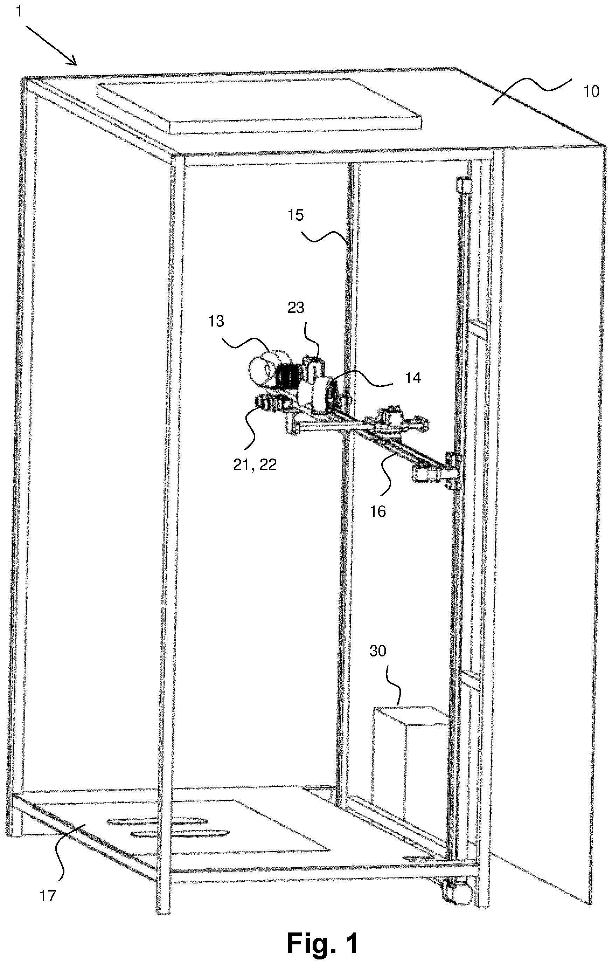

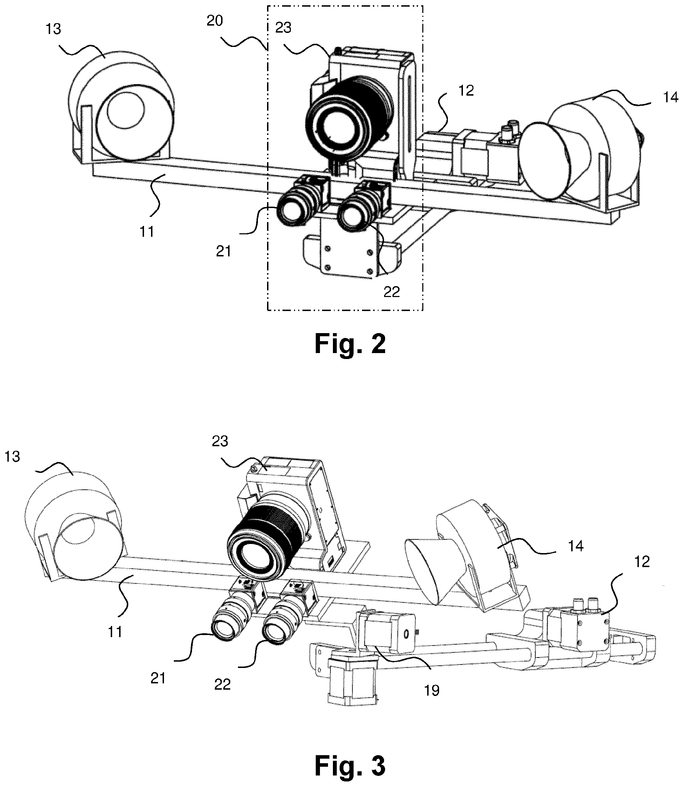

Figs. 1 and 2 show the proposed architecture of the invention for checking skin lesions, including primary and secondary skin lesions, according to an embodiment. In this particular embodiment, the dermatoscopy device 1 includes an enclosed chamber 10 designed to accommodate a user/patient to be examined; a guiding system 15 formed by two vertical guide members attached on an interior wall of the enclosed chamber 10 (not limitative as in other embodiments the guiding system can comprise a robotic arm or can be formed by guide members attached on different interior walls of the enclosed chamber 10); a displacement element 12 arranged on a guiding railway 16 of the cited guiding system 15 and suitable to be moved on the three axes (x, y, z) inside the enclosed chamber 10; an image acquisition system 20 attached on a support 11 of the displacement element 12; two lighting elements 13, 14; a positioning unit 17; and a computing unit 30 with one or more processors and at least one memory.

The computing unit 30 is operatively connected to the displacement element 12 and to the image acquisition system 20 and is adapted and configured to implement/execute different algorithms in order to control operation thereof. It should be noted that in other embodiments, the computing unit 30 instead of being included in the dermatoscopy device 1 can be located remote therefrom. For example, in this latter case, the computing unit can be a PC, a cloud server, a Smartphone, a Tablet, etc. placed distant from the dermatoscopy device 1.

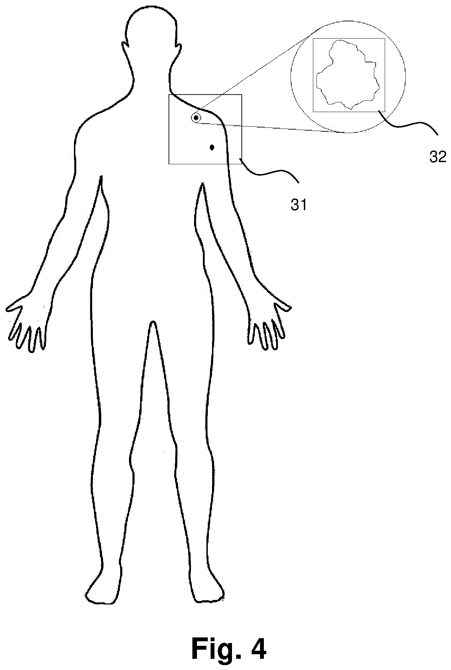

In the particular embodiment of Figs. 1 and 2, the image acquisition system 20 includes two cameras 21, 22 that are configured to operate as a stereoscopic camera (hence from now on being termed stereoscopic cameras) and a dermatoscopic camera 23. The two stereoscopic cameras 21, 22 operate simultaneously and regardless of the dermatoscopic camera 23. The dermatoscopic camera 23 is configured to acquire dermatoscopic images 32 (see Fig. 4 for an example) at a certain distance from the user, for example, at 100-1000 mm, particularly at 300-600 mm from the user.

In other embodiments (not showed), the image acquisition system 20 includes a single stereoscopic camera, for example consisting of a structured-light 3D sensor, or active stereo camera with a structured light source or a laser, a passive stereo camera, a TOF camera, etc.

In other embodiments (not showed either) the dermatoscopy device 1 includes a higher number of lighting elements, for example four or more lighting elements.

With regard to the lighting elements 13, 14, each one comprises a high-power LED, with a light confinement chamber and a refrigerating system (i.e. each LED element has a radiator and a fan attached). Moreover, each lighting element 13, 14 has a polarization filter having a 90° offset with respect to a polarization filter of the dermatoscopic camera 23. Thus, a cross polarization is generated.

The lighting elements 13, 14 are arranged on the cited support 11 separated from the image acquisition system 20 in order to operate at a given angle (between 35º and 55º) considering the operating distance of the dermatoscopic camera 23.

In the embodiment of Figs. 1 and 2, the positioning unit 17 comprises a floor display that is configured to indicate to the user a position in which (s)he has to be placed/located inside the enclosed chamber in order the images (31, 32) from different body sides being acquired. In this case, the user has to move herself/himself to the indicated position. In other embodiments, the positioning unit 17 can automatically move the user towards the displacement element 12 and image acquisition system 20.

Fig. 3 shows another embodiment in which the displacement element 12 includes an articulable element 19 that allows the additional movement of the displacement element 12 in multiple degrees of freedom. That is, with the articulable element 19 the displacement element 12 can be not only moved in relation to the (x, y, z) axis but can also be pivotable in relation to different rotation axis.

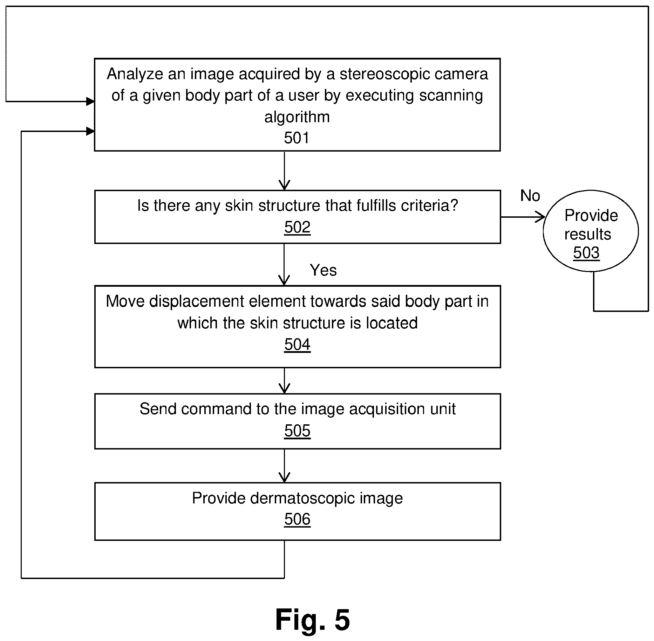

With reference now to Fig. 5, therein it is illustrated an embodiment of the processing steps executed by the computing unit. This method is initiated once a user to be examined (see Fig. 4 for an example) is accommodated within the enclosed chamber 10 of the proposed dermatoscopy device 1 and once the image acquisition system 20 is moved towards a first body part (e.g. the torso) of a given body side (e.g. the ventral side of the body) of the user. In this case it has been supposed that the image acquisition system 20 includes two cameras configured to operate as a passive stereoscopic camera (not limiting as other types of cameras can be used as indicated above) and a dermatoscopic camera.

At step 501, the computing unit analyzes the acquired stereoscopic image(s) 31 by executing a scanning algorithm that checks whether skin structures (e.g. actinic keratosis, moles, skin marks, etc.) included in said stereoscopic image(s) 31 fulfill a given criteria (step 502). If no skin structures fulfilling the criteria are found, the computing unit simply provides (step 503) the results of the previous analysis. Otherwise, i.e. if a given skin structure has been determined as fulfilling the given criteria, the computing unit, at step 504, executes a motion algorithm that automatically moves (and optionally rotates) the displacement element 12, and so the image acquisition system 20 and the lighting elements 13, 14, towards the body part where the skin structure is located. Then, at step 505, the computing unit triggers a command/order to the image acquisition unit 20 in order the dermatoscopic camera 23 acquiring, at a determined distance from the user, particularly at approx. 300-600 mm from the user, at least one dermatoscopic image 32 of said skin structure. Once the dermatoscopic image 32 is acquired, the computing unit, at step 506, provides the dermatoscopic image, for example via a display thereof, via a specific user interface, by printing it, etc. In this sense, a physician/doctor can latter use the dermatoscopic image to make an accurate diagnosis of the skin lesion.

It should be noted that, previous steps are performed for all the body sides of the user, if necessary. For example, for the lateral and dorsal sides of the user, and for different body parts, such as the legs, arms, head, back, etc., of each body side. If the user has to be moved to a given position in order the image/images being acquired from a specific body part or body side, a further step of the method could be an indication of such a position.

In certain embodiments, said criteria is based on an ABCD rule of dermatoscopy based on the criteria asymmetry, A, border, B, color, C, and differential structure, D, of the skin structures. Complementarily or alternatively, in certain embodiments, the scanning algorithm can comprise a neural network, a decision tree-based algorithm and/or a support vector machine.

In an embodiment, the scanning algorithm is executed/implemented on at least one clinical image and particularly also uses additional features of the skin lesion (for example, size and/or volume of the skin lesion). Then, the algorithm performs, at least, a classification task, and as a result, provides a value of "potential malignity" of a given lesion. The criteria can be then based on that value and a given threshold.

In other embodiments, in the particular case that two cameras 21, 22 configured to operate as a stereoscopic camera are used, the image(s) 31 acquired by each camera are analyzed in parallel. Once the scanning algorithm has detected a skin structure in one of the stereoscopic images 31, it matches the position thereof in the other stereoscopic image 31 in order to identify the exact position of the skin structure.

The scope of the present invention is defined in the following set of claims.

## Claims

1. A dermatoscopy device for checking skin lesions, comprising:
an enclosed chamber (10) to accommodate a user to be examined;
an image acquisition system (20) configured to acquire images from different body regions of said user;
lighting elements (13, 14); and
a computing unit (30), operatively connected to said displacement element (12) and to said image acquisition system (20), and configured to process said acquired images;
**characterized in that:**
the dermatoscopy device further comprises a displacement element (12) configured to be moved on axes x, y, z inside the enclosed chamber (10);
the image acquisition system (20) is attached on a support (11) of the displacement element (12) and comprises two different types of cameras, namely at least one camera of a first type (21, 22) and one camera of a second type (23), the at least one camera of the first type (21, 22) being configured to operate independently of the camera of the second type (23), the camera of the second type (23) being a dermatoscopic camera, and the at least one camera of the first type (21, 22) being a stereoscopic camera or two cameras configured to operate as a stereoscopic camera;
the lighting elements (13, 14) comprises at least two lighting elements each one attached on an extreme of the support (11) of the displacement element (12); and
the processing comprises:
• analyzing, for each body region, at least one image (31) acquired by the at least one camera of the first type (21, 22);
• executing a scanning algorithm that detects skin structures in the acquired image (31) and that checks whether the detected skin structures fulfill a given criteria;
• upon a given skin structure of a given body region is determined as fulfilling the given criteria, executing a motion algorithm that automatically moves the displacement element (12) to the given body region; and
• providing a triggering order to the image acquisition system (20), and as a result of the triggering order, the camera of the second type (23) acquiring, at a determined distance, at least one image (32) of the given skin structure, the distance being comprised in a range between 100-1000 mm from the user.

2. The device of claim 1, wherein the at least one camera of the first type (21, 22) comprises a passive stereo camera, an active stereo camera with a structured light source or a laser, a time-of-flight, TOF, camera, or the combination of a LIDAR and a single camera.

3. The device of any one of the previous claims, wherein the displacement element (12) is arranged on a guiding railway (16) of a guiding system (15), the guiding system (15) being arranged on an interior wall of the enclosed chamber (10).

4. The device of any one of the previous claims, wherein each of the lighting elements (13, 14) comprises a light emitting diode, LED, with a light confinement chamber and a polarization filter, wherein said polarization filter has a 90°offset with respect to a polarization filter of the dermatoscopic camera (23).

5. The device of any one of the previous claims, further comprising a positioning unit (17) configured to indicate a position in which the user has to be placed inside the enclosed chamber (10).

6. The device of claim 3, further comprising a positioning unit (17) configured to automatically move the user towards said interior wall of the enclosed chamber (10).

7. The device of any one of the previous claims, wherein the displacement element (12) further comprises at least one articulable element (19) to allow the displacement element (12) to be moved in multiple degrees of freedom.

8. The device of any one of the previous claims, wherein the criteria is based on an ABCD rule of dermatoscopy based on the criteria asymmetry, A, border, B, color, C, and differential structure, D, of the skin structures.

9. The device of claim 1, wherein the distance is comprised in a range between 300-600 mm.

## Patentansprüche

1. Dermatoskopievorrichtung zur Überprüfung von Hautläsionen, Folgendes umfassend:
eine geschlossene Kammer (10) zur Aufnahme eines zu untersuchenden Benutzers;
ein Bilderfassungssystem (20), das so ausgebildet ist, dass es Bilder von verschiedenen Körperregionen des Benutzers erfasst;
Beleuchtungselemente (13, 14); und
eine Recheneinheit (30), die operativ mit dem Verschiebungselement (12) und mit dem Bilderfassungssystem (20) verbunden ist und so ausgebildet ist, dass sie die erfassten Bilder verarbeitet;
**dadurch gekennzeichnet, dass**:
die Dermatoskopievorrichtung ferner ein Verschiebungselement (12) umfasst, das so ausgebildet ist, dass es auf den Achsen x, y, z innerhalb der geschlossenen Kammer (10) bewegt werden kann;
das Bilderfassungssystem (20) an einem Träger (11) des Verschiebungselements (12) angebracht ist und zwei verschiedene Arten von Kameras umfasst, und zwar mindestens eine Kamera eines ersten Typs (21, 22) und eine Kamera eines zweiten Typs (23), wobei die mindestens eine Kamera des ersten Typs (21, 22) so ausgebildet ist, dass sie unabhängig von der Kamera des zweiten Typs (23) arbeitet, wobei die Kamera des zweiten Typs (23) eine dermatoskopische Kamera ist und die mindestens eine Kamera des ersten Typs (21, 22) eine stereoskopische Kamera ist bzw. zwei Kameras, die so ausgebildet sind, dass sie als eine stereoskopische Kamera arbeiten;
die Beleuchtungselemente (13, 14) mindestens zwei Beleuchtungselemente umfassen, von denen jedes an einem Ende des Trägers (11) des Verschiebungselements (12) angebracht ist; und
die Behandlung Folgendes umfasst:
• Analysieren mindestens eines Bildes (31), das von der mindestens einen Kamera des ersten Typs (21, 22) erfasst wurde, für jede Körperregion;
• Ausführen eines Scan-Algorithmus, der Hautstrukturen in dem erfassten Bild (31) erkennt und überprüft, ob die erkannten Hautstrukturen ein vorgegebenes Kriterium erfüllen;
• Ausführen eines Bewegungsalgorithmus, der das Verschiebungselement (12) automatisch zu der gegebenen Körperregion bewegt, wenn eine gegebene Hautstruktur einer gegebenen Körperregion als die gegebenen Kriterien erfüllend bestimmt wird; und
• Bereitstellen eines Auslösebefehls an das Bilderfassungssystem (20), und als Ergebnis des Auslösebefehls Erfassen von mindestens einem Bild (32) der gegebenen Hautstruktur durch die Kamera des zweiten Typs (23) in einem bestimmten Abstand, wobei der Abstand einen Bereich zwischen 100-1000 mm vom Benutzer umfasst.

2. Vorrichtung nach Anspruch 1, wobei die mindestens eine Kamera des ersten Typs (21, 22) eine passive Stereokamera, eine aktive Stereokamera mit einer strukturierten Lichtquelle oder einem Laser, eine Time-of-Flight-(TOF)-Kamera oder die Kombination aus einem LIDAR und einer einzelnen Kamera umfasst.

3. Vorrichtung nach einem der vorhergehenden Ansprüche, wobei das Verschiebungselement (12) an einer Führungsschiene (16) eines Führungssystems (15) angeordnet ist, wobei das Führungssystem (15) an einer Innenwand der geschlossenen Kammer (10) angeordnet ist.

4. Vorrichtung nach einem der vorhergehenden Ansprüche, wobei jedes der Beleuchtungselemente (13, 14) eine Leuchtdiode (LED) mit einer Lichteinschlusskammer und einem Polarisationsfilter umfasst, wobei der Polarisationsfilter einen 90°-Versatz in Bezug auf einen Polarisationsfilter der dermatoskopischen Kamera (23) aufweist.

5. Vorrichtung nach einem der vorhergehenden Ansprüche, die ferner eine Positionierungseinheit (17) umfasst, die so ausgebildet ist, dass sie eine Position angibt, in der der Benutzer innerhalb der geschlossenen Kammer (10) zu positionieren ist.

6. Vorrichtung nach Anspruch 3, die ferner eine Positionierungseinheit (17) umfasst, die so ausgebildet ist, dass sie den Benutzer automatisch in Richtung der Innenwand der geschlossenen Kammer (10) bewegt.

7. Vorrichtung nach einem der vorhergehenden Ansprüche, wobei das Verschiebungselement (12) ferner mindestens ein gelenkiges Element (19) umfasst, so dass das Verschiebungselement (12) in mehreren Freiheitsgraden bewegt werden kann.

8. Vorrichtung nach einem der vorhergehenden Ansprüche, wobei die Kriterien auf einer ABCD-Regel der Dermatoskopie basieren, die auf den Kriterien Asymmetrie, A, Begrenzung, B, Colorit (Farbe), C, und Differentialstruktur, D, der Hautstrukturen beruht.

9. Vorrichtung nach Anspruch 1, wobei der Abstand einen Bereich zwischen 300-600 mm umfasst.

## Revendications

1. Un dispositif de dermascopie pour la vérification de lésions cutanées, comportant :
une chambre fermée (10) pour loger un utilisateur à examiner ;
un système d'acquisition d'images (20) configuré pour acquérir des images de diverses régions du corps de cet utilisateur ;
des éléments d'éclairage (13,14) ; et
une unité informatique (30), fonctionnellement connectée à cet élément de déplacement (12) et à ce système d'acquisition d'images (20), et configurée pour traiter ces images acquises ;
**caractérisé en ce que :**
le dispositif de dermascopie comporte en outre un élément de déplacement (12) configuré pour être déplacé sur des axes x, y, z à l'intérieur de la chambre fermée (10) ;
le système d'acquisition d'images (20) est attaché sur un support (11) de l'élément de déplacement (12) et comporte deux types différents de caméras, notamment au moins une caméra d'un premier type (21, 22) et une caméra d'un deuxième type (23), la au moins une caméra du premier type (21, 22) étant configurée pour fonctionner indépendamment de la caméra du deuxième type (23), la caméra de deuxième type (23) étant une caméra dermoscopique , et la au moins une caméra du premier type (21, 22) étant une caméra stéréoscopique ou deux caméras configurées pour fonctionner comme caméra stéréoscopique ;
les éléments d'éclairage (13, 14) comportent au moins deux éléments d'éclairage chacun étant attaché sur une extrémité du support (11) de l'élément de déplacement (12) ; et
le traitement comporte :
• analyser, pour chaque région du corps, au moins une image (31) acquise par au moins une caméra du premier type (21, 22) ;
• exécuter un algorithme de numérisation qui détecte des structures cutanées dans l'image acquise (31) et qui vérifie si les structures cutanées détectées remplissent un certain critère ;
• après qu'il est déterminé qu'une certaine structure cutanée d'une certaine région du corps remplit ce critère, exécuter un algorithme de mouvement qui déplace automatiquement l'élément de déplacement (12) à une certaine région du corps ; et
• fournir un ordre de déclenchement au système d'acquisition d'images (20), et suite à l'ordre de déclenchement, la caméra du deuxième type (23) acquérant, à une certaine distance, au moins une image (32) de cette structure cutanée, la distance étant comprise dans une fourchette entre 100 et 1.000 mm de l'utilisateur.

2. Le dispositif de la revendication 1, où la au moins une caméra du premier type (21, 22) comporte une caméra stéréo passive, une caméra stéréo active ayant une source de lumière structurée ou une caméra laser, de temps de vol, TOF ou la combinaison d'une LIDAR et d'une seule caméra.

3. Le dispositif conformément à une quelconque des revendications précédentes, où l'élément de déplacement (12) est aménagé sur un rail de guidage (16) d'un système de guidage (15), le système de guidage (15) étant aménagé sur une paroi intérieure de la chambre fermée (10).

4. Le dispositif conformément à une quelconque des revendications précédentes, où chacun des éléments d'éclairage (13, 14) comporte une diode électroluminiscente, LED, ayant une chambre de confinement de lumière et un filtre de polarisation, où ce filtre polarisation a un décalage de 90º par rapport à un filtre de polarisation de la caméra dermascopique (23).

5. Le dispositif conformément à une quelconque des revendications précédentes, comportant en outre une unité de positionnement (17) configurée pour indiquer une position dans laquelle l'utilisateur doit être placé à 'intérieur de la chambre fermée (10).

6. Le dispositif de la revendication 3, comportant en outre une unité de positionnement (17) configurée pour déplacer automatiquement l'utilisateur vers cette paroi intérieure de la chambre fermée (10).

7. Le dispositif conformément à une quelconque des revendications précédentes, où l'élément de déplacement (12) comporte en outre au moins un élément articulable (19) pour permettre à l'élément de déplacement (12) d'être déplacé dans de multiples degrés de liberté.

8. Le dispositif conformément à une quelconque des revendications précédentes, où le critère est basé sur une règle ABCD de dermoscopie basée sur l'asymétrie des critères, A, bord, B, couleur, C, et structure différentielle, D, des structures cutanées.

9. Le dispositif de la revendication 1, où la distance est comprise dans une fourchette entre 300 et 600 mm.
